# EUROPÄISCHE PATENTSCHRIFT

(11) **EP 3 294 157 B1**
(45) Veröffentlichungstag und Bekanntmachung des Hinweises auf die Patenterteilung: **13.02.2019**
(21) Anmeldenummer: 17729455.0
(22) Anmeldetag: 08.06.2017
(51) Int. Cl.: A61B 17/28

(54) **CHIRURGISCHES INSTRUMENT MIT ABSTANDHALTENDEM SCHWENKELEMENT**
SURGICAL INSTRUMENT HAVING A SPACING PIVOTING ELEMENT
INSTRUMENT CHIRURGICAL PRÉSENTANT UN ÉLÉMENT PIVOTANT DE CONSERVATION DE LA DISTANCE

(30) Priorität: 16.06.2016 DE 102016111001
(43) Veröffentlichungstag der Anmeldung: 21.03.2018
(73) Patentinhaber: Aesculap AG, 78532 Tuttlingen (DE)
(72) Erfinder: BARTHELMES, Sven, 78576 Emmingen-Liptingen (DE); WEISSHAUPT, Dieter, 78194 Immendingen (DE); MORALES, Pedro, 78532 Tuttlingen (DE); MORALES, Daniel, 78532 Tuttlingen (DE)
(74) Vertreter: Winter, Brandl, Fürniss, Hübner, Röss, Kaiser, Polte - Partnerschaft mbB
(86) Internationale Anmeldenummer: PCT/EP2017/064016
(87) Internationale Veröffentlichungsnummer: WO 2017/216039

(56) Entgegenhaltungen:
- EP-A1- 2 594 210
- EP-A1- 2 873 381
- DE-A1- 10 101 425
- US-A- 2 305 156

## Beschreibung

Die vorliegende Erfindung betrifft ein chirurgisches Instrument mit zwei relativ zueinander verschwenkbaren Instrumententeile gemäß dem Oberbegriff des Anspruchs 1. Bei chirurgischen Instrumenten der erfindungsgemäßen Art kann es sich beispielsweise um vorwiegend in der Chirurgie eingesetzte Klemmen, Greifvorrichtungen, Schneidvorrichtung und/oder Haltevorrichtungen mit beliebiger Geometrie handeln insbesondere Scheren, Fasszangen und dergleichen Brancheninstrumenten.

### Hintergrund der Erfindung

Chirurgische Instrumente der Instrumentenbranchen-Bauart, die eine (scherenartige) Relativbewegung zweier schwenkbar aneinander scharnierter Instrumententeile gewährleisten, finden auf nahezu allen medizinischen Gebieten Anwendung. Um eine ebene Schwenk-Führung der Instrumententeile zueinander zu ermöglichen, weisen die Instrumententeile Berührflächen auf, die bei einer Relativverschwenkung unter Reibung aufeinander abgleiten und so ein Verkanten der Instrumententeile verhindern. Da die beiden Instrumententeile in der Regel aus dem gleichen Werkstoff gefertigt sind, besteht jedoch die Gefahr des Festfressens der Instrumententeile im Scharnierbereich. Diese Gefahr wird von Fertigungstoleranzen der einzelnen Instrumententeile, die im zusammengebauten Zustand zu unterschiedlichen Berührflächen führen, noch weiter verschärft. Der daraus folgende Materialverschleiß, ebenso wie eine durch Reibung hervorgerufene Kaltverschweißung, verringern die Lebensdauer von chirurgischen Instrumenten. Zusätzlich wird eine Bedienung eines solchen chirurgischen Instruments, die den höchsten Anforderungen der Präzision unterliegt, durch die vorstehende Problematik erschwert.

### Stand der Technik

Es existieren verschiedene Dokumente, die sich mit aufeinander reibenden Flächen von chirurgischen Instrumenten beschäftigen. So offenbart die DE 10101425 A1 ein chirurgisches Instrument mit zwei zueinander verschwenkbaren Instrumententeilen. Das Problem, das jener Anmeldung zugrunde liegt, ist, dass eine Reinigung und Sterilisation von Anlageflächen aufgrund der eingeschränkten Zugänglichkeit erschwert möglich ist. Um diese Zugänglichkeit zu verbessern, sind in jener Offenlegungsschrift die Instrumententeile konstruktiv derart modifiziert, dass sie außerhalb ihres normalen Arbeitsbereichs, der sich auf übliche Winkelspannen der Verschwenkung bezieht, so zueinander relativ bewegbar sind, dass flächige Anlagen der beiden Instrumententeile bereichsweise aufgehoben werden. Somit ist eine verbesserte Reinigung ermöglicht. Da auch die in jenem Dokument angeführten technischen Besonderheiten in einem Verschwenkbereich aufeinander reibende Flächen nach sich ziehen, kann das Problem des Festfressens hiermit nicht vermieden werden.

Ein weiteres gattungsgemäßes Dokument stellt die EP 2 594 210 A1 dar. Hierin ist ein medizinisches Instrument offenbart, in dem zwei Instrumententeile über einen Verbindungszapfen (Schwenkstift) verbunden sind. Eine Quernut in einer der beiden Instrumententeile bewirkt, dass die Instrumententeile in einem Zustand, in dem das Instrument nicht aktiv genutzt wird, relativ zueinander mit einem gewissen Spielraum, der die Reibung minimiert, beweglich sind. Dadurch, dass jener Spielraum nur dann auftritt, wenn das Instrument nicht aktiv genutzt wird, verringert dieser die Gefahr des Festfressens durch häufige Benutzung nicht.

Ein drittes Dokument sei mittels der DE 20 2010 010 843 U1 eingeführt. Auch hierin ist ein chirurgisches Instrument mit zwei relativ zueinander verschwenkbaren Instrumententeile offenbart, die über eine flächige Reibung der Gefahr des Festfressens ausgesetzt sind. Der darin beschriebene Mechanismus zur Verringerung jener Reibung bezieht sich wiederum nur auf einen Zustand, in dem das Instrument nicht aktiv genutzt ist, weshalb auch auf diese Weise ein Festfressen nicht zuverlässig vermieden werden kann.

Letztlich offenbart die EP 2 873 381 A1 ein gattungsgemäßes Instrument gemäß dem Oberbegriff des Patentanspruchs 1.

### Kurzbeschreibung der Erfindung

Der vorliegenden Erfindung liegt angesichts dieses Standes der Technik die Aufgabe zugrunde, ein chirurgisches Instrument der Branchen-Bauart bereitzustellen, bei dem zwei Instrumententeile in einem Koppel-/Scharnierbereich, also einem Bereich, in dem zwei in diesem Bereich flächige Komponenten (Branchen) zueinander verschwenkt werden, derart zueinander ausrichtet werden, dass diese stabil und robust geführt sind (um ein Verkanten der Instrumententeile zu vermeiden), ohne dabei große Auflageflächen aufzuweisen, um einem Festfressen der einzelnen Instrumententeile vorzubeugen.

Diese Aufgabe wird mittels eines chirurgischen Instruments mit den Merkmalen des Anspruchs 1 gelöst. Vorteilhafte Ausgestaltungen sind Gegenstand der Unteransprüche.

Aus dieser erfindungsgemäßen Ausgestaltung der (Ober-)Seiten des Koppel-/Scharnierbereichs gemäß dem Patentanspruch 1 lassen sich somit beispielsweise folgende Vorteile ableiten:
- Ein Festfressen der einzelnen Instrumententeile zueinander wird vermieden, wodurch die Lebensdauer des chirurgischen Instruments steigt.
- Mittels der vorbestimmten und gegenüber dem Stand der Technik verringerten Auflageflächen der einzelnen Instrumententeile, die deutlich verringerten Toleranzschwankungen unterliegen, ist der Öffnungswiderstand reduziert / der Gang des Instruments verbessert und über eine Produktkette deren Lebensdauer hin konstant.
- Ein Schmierprozess des Instruments kann zeiteffizienter durchgeführt werden, da das Bedürfnis an Schmiermedium pro Instrument aufgrund der gegenüber dem bekannten Stand der Technik kleiner werdenden Auflageflächen einheitlicher wird. Somit wird eine effiziente maschinelle Schmierung des Instruments beispielsweise im Rahmen eines Desinfektionsprozesses ermöglicht.
- Zusätzlich ist die Desinfizierbarkeit dadurch optimiert, dass die Auflageflächen im Koppel-/Scharnierbereich besser zugänglich und zudem kleiner sind.
- Durch die vorstehend erwähnte Verbesserung des Gangs des Instruments ist weiterhin zum einen die durch einen chirurgischen Eingriff erreichbare Präzision und zum anderen der Bedienkomfort für den Operateur erhöht.

Der Gegenstand der vorliegenden Erfindung betrifft demnach zusammengefasst ein chirurgisches Instrument mit zwei scherenartig miteinander gekoppelten (aneinander scharnierten) Instrumententeile, die im Bereich Ihrer Scharnierverbindung/Kopplung jeweils zumindest eine vorzugsweise flächige/ebene Anlage-/Berührseite ausbilden, welche weiter vorzugsweise auch den maximalen Öffnungswinkel der beiden Instrumentenbrachen definieren/begrenzen und an denen die beiden Instrumententeile im montierten Zustand gleitend aneinander liegen.

Gemäß einem ersten, ggf. unabhängig zu beanspruchenden Aspekt der vorliegenden Erfindung ist zumindest eine Anlage-/Berührseite der einen oder beider Instrumententeile mit einer flächigen Materialabtragung im näheren Bereich zu der Scharnierverbindung/Kopplung versehen, derart, dass sich in diesem Bereich eine (ebene) Senke und im entfernteren Bereich zu der Scharnierverbindung/Kopplung der Scharnierverbindung/Kopplung zumindest eine (ebene) Erhebung ausbildet. Die Erhebung(en) bildet (bilden) dabei die Gleitfläche für das gegenüberliegende Instrumententeil.

Gemäß einem anderen, ggf. unabhängig zu beanspruchenden Aspekt der vorliegenden Erfindung besteht die Scharnierverbindung/Kopplung aus einem stiftartigen Schwenkelement bzw Scharnierbolzen mit einem gegenüber seinen axialen Endabschnitten verdickten/radial aufgeweiteten Mittelabschnitt oder einem auf den Mittelabschnitt aufgeschobenen Ring/Hülse, wodurch sich in beiden Fällen zumindest ein, vorzugsweise zwei axial beabstandete Axialanschläge/Auflagekanten ausbilden. Vorzugsweise ist der Axialabstand der beiden Axialanschläge so bemessen, dass sich zwischen den Anlage-/Berührseiten der beiden Instrumententeile in (unbetätigter) Konstruktionslage ein (Mikro-)Spalt ergibt, derart, dass sich bei einer Schwenkbetätigung der beiden aneinander scharnierten Instrumententeile die Gleit-Auflagekraft zwischen den Instrumententeilen gegenüber dem Stand der Technik verringert.

Konkreter ausgedrückt hat das chirurgische Instrument der vorliegenden Erfindung bevorzugt ein erstes Instrumententeil (weibliches Instrumententeil), das in einem Koppel-/Scharnierbereich ein vorzugsweise kastenförmiges Hohlvolumen (Durchsteckkasten) definiert, und ein zweites Instrumententeil (männliches Instrumententeil), das im Koppel-/Scharnierbereich einen Führungsabschnitt (Durchsteckabschnitt) ausbildet. Dieser Führungsabschnitt ist dazu vorbereitet, das Hohlvolumen zumindest teilweise zu durchlaufen (Ausbildung eines sogenannten Durchsteckschlusses). Weiterhin weist das chirurgische Instrument ein Schwenkelement (Scharnierbolzen) mit einem oberen Endabschnitt, einem Mittelabschnitt und einem unteren Endabschnitt auf, das das weibliche Instrumententeil und das in das Hohlvolumen des weiblichen Instrumententeils eingesteckte männliche Instrumententeil im Bereich des Hohlvolumens und des Führungsabschnitts derart miteinander koppelt, dass diese um eine Rotationsachse des Schwenkelements relativ zueinander verschwenkbar sind. Auf diese Weise kommt der Durchsteckschluss zwischen den beiden Instrumententeile zustande, der eine Relativverschwenkung bei ebener Führung der Branchen zueinander zulässt.

Weiter bevorzugst ist zwischen dem oberen Abschnitt und dem Mittelabschnitt des Schwenkelements eine radial umlaufende obere Anlagestufe/Anlagekante sowie zwischen dem unteren Abschnitt und dem Mittelabschnitt des Schwenkelements eine radial umlaufende untere Anlagestufe/Anlagekante ausgebildet. Diese Anlagestufen realisieren eine Abstandshaltefunktion des Schwenkelements, denn die radial umlaufenden Stufen/Vorsprünge ermöglichen zumindest eine Anlagefläche bzw. einen Anschlag für Innenflächen des Hohlvolumens des weiblichen Instrumententeils. Somit wird ein gleichbleibender (Parallel-)Abstand zweier sich gegenüberliegender Innenflächen des Hohlvolumens bewirkt, zwischen den der Führungsabschnitt des männlichen Instrumententeils gleitend gehalten ist, was eine robuste und präzise Führung der beiden Instrumententeile relativ zueinander ermöglicht, jedoch einem Festfressen beider Instrumententeile vorbeugt infolge zu hoher Aufstandskräfte zwischen den beiden Innenflächen des kastenförmigen Hohlvolumens des weiblichen Instrumententeils und dem Führungsabschnitt des männlichen Instrumententeils.

Weiterhin von Vorteil ist es, wenn die obere Anlagestufe dazu vorbereitet ist, eine obere Innenfläche des Hohlvolumens zu kontaktieren und/oder die untere Anlagestufe dazu vorbereitet ist eine untere Innenfläche des Hohlvolumens zu kontaktieren. Somit bildet die obere und/oder die untere Stufe eine geometrisch vorbestimmte Anlagefläche für die jeweiligen Innenflächen des Hohlvolumens. Diese Flächen der Anlagestufen sind vorzugsweise Teil des Mittelabschnitts des Schwenkelements und können derart ausgestaltet / bearbeitet sein, dass Berührflächen im Koppel-/Scharnierbereich zwischen den beiden Branchen ausreichend groß für eine robuste Führung und zugleich ausreichend klein sind, um ein Festfressen zu vermeiden.

Sobald der Führungsabschnitt des männlichen Instrumententeiles zumindest die eine Vertiefung/Senke aufweist, die zumindest einen Spalt zwischen dem männlichen Instrumententeil und dem weiblichen Instrumententeil bewirkt, wie dies vorstehend bereits allgemein beschrieben wurde, verstärkt dies die erfindungsgemäßen Vorteile. Jener Spalt bewirkt, dass zwischen dem Hohlvolumen bzw. den beiden parallel beanstandeten, zur Gleitführung vorbestimmten Innenflächen des Hohlvolumens und dem Führungsabschnitt bzw. den beiden voneinander abgewandten, zur Gleitführung vorbestimmten Anlageseiten zumindest an der Anlageseite mit darauf ausgebildeter Vertiefung/Senke nur im Bereich der Erhebungen eine Kontakt- / Berührfläche, die hierin als Führungsflächen bezeichnet sind, definiert wird. Die Senke/Vertiefung ist hinsichtlich ihrer Geometrie und ihrer Tiefe frei und variabel ausbildbar, kann aber bevorzugt so definiert werden, dass der dadurch erzeugte Spalt zwischen dem Führungsabschnitt des männlichen Instrumententeils und der zugeordneten Innenfläche des Hohlvolumens des weiblichen Instrumententeils das Eindringen von Desinfektionsmittel begünstigt. Es ist hierbei vorteilhaft, wenn die Tiefe der Senke bezüglich der jeweiligen Erhebungen überall konstant ist.

Es ist vorteilhaft, wenn die Axialabmessung des Mittelabschnitts des stiftförmigen Schwenkelements und die Senkentiefe so aufeinander abgestimmt sind, dass die vom radial aufgeweiteten Mittelabschnitt des Schwenkelements oder vom aufgesteckten Ring/Hülse gebildeten Anlage-/Anschlagsstufen auch weiterhin ihre Anschlags- bzw. Abstandshaltefunktion beibehalten, wie diese vorstehend beschrieben wurde, d. h. sich auf Höher der Erhebungen anordnen.

Weiter vorteilhaft wäre es, wenn sich die zumindest eine Senke/Vertiefung über die gesamte Führungsabschnittsbreite des männlichen Instrumententeils erstreckt (ohne Rahmenbildung), was sich besonders günstig auf die Vermeidung des Festfressens und auch auf die Desinfizierbarkeit auswirkt.

Wenn der Bereich des Führungsabschnitts distal von der Senke/Vertiefung zumindest eine vordere Führungsfläche (Erhebung) ausbildet, die dazu vorbereitet ist, mit zumindest einer der Innenflächen des Hohlvolumens in Kontakt zu stehen, und der Bereich des Führungsabschnitts proximal von der Senke/Vertiefung zumindest eine hintere Führungsfläche (Erhebung) ausbildet, die dazu vorbereitet ist, mit zumindest einer der Innenflächen des Hohlvolumens in Kontakt zu stehen, folgen daraus weitere Vorteile. So ist nicht nur die Vertiefungsgeometrie, sondern auch die der Führungsflächen/Erhebungen flexibel und anpassbar. Dabei sei an dieser Stelle unbedingt darauf hinzuweisen, dass die Senke/Vertiefung und die daraus sich ergebenden Erhebungen gemäß vorstehender Beschreibung nicht nur an einer Auflageseite des männlichen Instrumententeils ausgebildet sein muss, sondern auch (spiegelbildlich) auf der anderen, abgewandten Auflageseite des männlichen Instrumententeils zusätzlich ausgebildet sein kann. Dabei ist die Geometrie beider Senken/Vertiefungen identisch zueinander oder unterschiedlich.

Demnach ist die/jede Auflageseite jeweils bestehend aus Senke und beidseits der Senke daraus resultierenden Erhebungen im gesamten Koppel-/Scharnierbereich variabel gestaltbar. Es kontaktieren sich das männliche Instrumententeil und das weibliche Instrumententeil nur in den Bereichen der Erhebungen, die von der Geometrie der Vertiefung teilweise bestimmt sind. Der weitere Kontakt, der für eine gleichmäßige Führung der Verschwenkung notwendig ist, wird von dem Mittelabschnitt bzw. den Stufen des Schwenkelements und den Innenseiten des Hohlvolumens realisiert.

Entlang einer Längsachse des Instruments, die von einem distalen Griffbereich zu einem proximalen Instrumenten-Eingriffsbereich (Maulteil) verläuft, der von den Instrumenten-Branchen gebildet wird, bildet sich demnach an drei Stellen / Abschnitten der Oberseite und an drei Stellen / Abschnitten der Unterseite des männlichen Instrumenten-Teils eine Berühr-Gleitfläche zwischen dem männlichen und weiblichen Instrumententeil aus: an der vorderen/distalen Erhebung, an der jeweiligen axialen Stufe des Schwenkelements und an der hinteren/proximalen Erhebung der beiden Anlageseiten des männlichen Instrumententeils.

Es kann die Senke/Vertiefung als Einfräsung, die quer zu der Längsachse des chirurgischen Instruments verläuft, in den Führungsabschnitt eingebracht werden, wodurch die Herstellung des männlichen Instrumententeils kosten- und zeiteffizient ist. Weiterhin können somit Fertigungsschwankungen hinsichtlich der Toleranz minimal gehalten werden.

Sobald das bolzen-/stiftförmige Schwenkelement einteilig ausgestaltet ist und der obere (End-)Abschnitt des Schwenkelements und der untere (End-)Abschnitt des Schwenkelements zylindrisch ausgeformt sind, lässt sich die erfindungsgemäße Aufgabe des Schwenkelements, nämlich ein reibungsarmes Verschwenken der Instrumententeile zueinander bei gleichzeitiger Abstandshalterung des Hohlvolumens, besonders gut realisieren.

Auch eine zweiteilige Ausgestaltung des Schwenkelements, bei der der obere (End-)Abschnitt des Schwenkelements und der untere (End-)Abschnitt des Schwenkelements zylindrisch ausgeformt sind, wohingegen der radial aufgeweitete Mittelabschnitt durch eine aufgeschobene Hülse ausgebildet wird, ist als vorteilhafte Ausführungsform Teil der Erfindung. Bei einem zweiteiligen Schwenkelement bietet es sich an, dass die beiden Teile aus unterschiedlichen Materialien gefertigt sind. Der obere und der untere (End-) Abschnitt können einteilig als rotationssymmetrischer Stift/Bolzen und der Mittelabschnitt als Ring/Hülse mit einer Öffnung, durch die der Stift greift, ausgestaltet sein. Eine Werkstoffkombination von Stift und Ring beispielsweise aus Keramik und Austenit ist denkbar.

Von Vorteil wäre zudem, wenn der Mittelabschnitt des Schwenkelements zylindrisch ausgestaltet ist. Somit bildet er einen Flächenkontakt mit einer Öffnung im Führungsabschnitt des männlichen Instrumententeils aus, was eine zusätzlich robuste Führung nach sich zieht. Zudem ist das Schwenkelement somit als Stufenniet ausgestaltbar, was zuverlässig und kostengünstig herstellbar ist.

Auch eine sphärische / ballische Ausgestaltung des Mittelabschnitts des Schwenkelements ist möglich. Diese bringt Vorteile bei der Reinigung / Schmierung mit sich, da infolge der ausgebildeten zumindest einen Senke/Vertiefung im Führungsabschnitt des männlichen Instrumententeils mittels des sphärischen Mittelabschnitts ein dreidimensionales Öffnen der beiden Instrumententeile möglich ist, insbesondere dann, wenn das chirurgische Instrument um ca. 70° bis 110°, vorzugsweise ca. 90° aufgeschwenkt ist. Somit ist die Beweglichkeit / Flexibilität des Instruments zumindest im Bereich dieses Aufschwenkwinkels erhöht, was sich positiv auf deren grundsätzliche Handhabung und somit viele Aspekte auswirkt.

Bei dem Schwenkelement kann es sich vorzugsweise um einen Niet handeln. Ebenfalls vorteilhaft ist es hierbei, das Schwenkelement und die Branchen des Instruments aus zueinander verschiedenen Materialien zu fertigen. Als Beispiel sei die Materialkombination X40Cr13/X20Cr13 genannt, wobei dies nicht als einschränkend anzusehen ist.

Bei der Senke/Vertiefung des Führungsabschnitts des männlichen Instrumententeils ist zu beachten, dass die Führungsflächen, welche in Kontakt mit dem kastenförmigen Hohlvolumen des weiblichen Instrumententeils stehen, ausreichend groß sind. Eine Verschwenken der Instrumententeile (männliches und weibliches Instrumententeil) von bis zu 60° zueinander ist bei der erfindungsgemäßen Größe der Führungsflächen bei robuster und präziser Führung ohne weiteres möglich.

Die Begriffe "oben" und "unten" im Rahmen dieser Anmeldung sind unter Bezug auf die nachfolgenden Zeichnungen zu verstehen, in denen sie mit Bezugszeichen versehen sind. Selbstverständlich kann bei einer Drehung des Instruments eine obere Fläche nach unten zeigen oder andersherum. Sie dienen demnach lediglich der Abgrenzung der Flächen voneinander und besitzen keine geometrische Gültigkeit in einem absoluten Bezugssystem.

### Figurenbeschreibung

Die Erfindung wird nachstehend anhand eines bevorzugten Ausführungsbeispiels unter Bezugnahme auf die begleitenden Figuren näher erläutert. Diese zeigen:

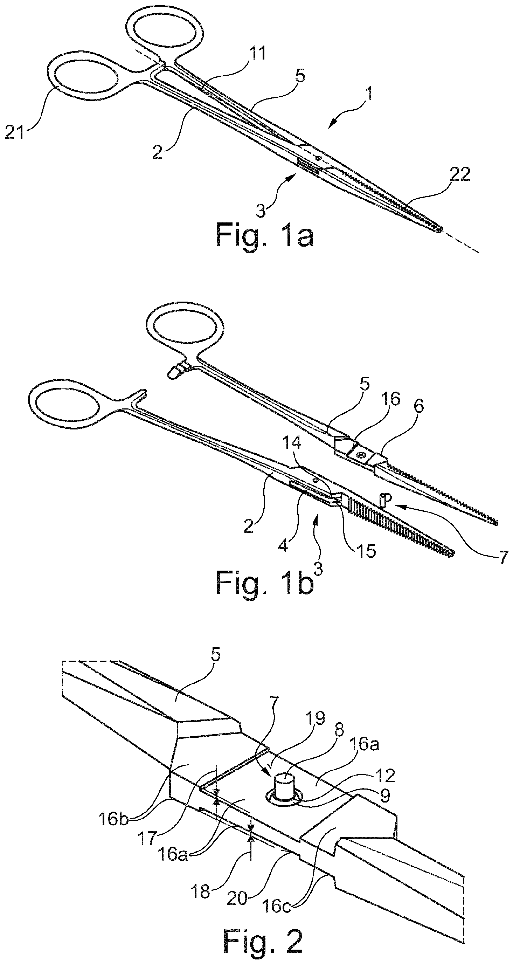

- Fig. 1a: eine perspektivische Ansicht eines chirurgischen Instruments gemäß einem bevorzugten Ausführungsbeispiel der vorliegenden Erfindung in einem zusammengebauten Zustand;
- Fig. 1: b eine Explosionszeichnung des chirurgischen Instruments aus Fig. 1a;
- Fig. 2: eine vergrößerte Ansicht eines Koppel-/Scharnierbereichs eines männlichen Instrumententeils des chirurgischen Instrumentes aus Fig. 1a, in der ein Schwenkelement (Schwenkstift) angeordnet ist;
- Fig. 3: ein Schwenkelement/Scharnierstift/Scharnierbolzen in einer ersten Ausführungsform gemäß der vorliegenden Erfindung;
- Fig. 4: ein Schwenkelement/Scharnierstift/Scharnierbolzen in einer zweiten Ausführungsform der vorliegenden Erfindung; und
- Fig. 5: ein Schwenkelement/Scharnierstift/Scharnierbolzen in einer dritten Ausführungsform der vorliegenden Erfindung.

Die Figuren sind lediglich schematischer Natur und dienen ausschließlich dem Verständnis der Erfindung. Die gleichen Elemente sind mit denselben Bezugszeichen versehen und können untereinander ausgetauscht werden.

Fig. 1a zeigt ein chirurgisches Instrument 1 der Branchenbauart gemäß der Erfindung in einem zusammengebauten sowie geschlossenem/unbetätigten Zustand (Konstruktionslage). Ein weibliches Instrumententeil bzw. erste Instrumententeil 2 ist über einen Koppel-/Scharnierbereich 3 derart mit einem männlichen Instrumententeil bzw. zweite Instrumententeil 5 gekoppelt / aneinander scharniert, dass diese zu einander verschwenkbar sind. Der Koppelbereich 3 wird von Seiten des ersten Instrumententeils 2 über ein Hohlvolumen (hohlvolumiger Durchsteckkasten) 4 und von Seiten des zweiten Instrumententeils 5 über einen Führungsabschnitt (Durchsteckabschnitt) 6 ausgebildet. Hierbei handelt es sich vorzugsweise um einen Durchsteck- / Kastenschluss, wie er beispielsweise aus der DE 20 2011 000800 U1 bekannt ist, sodass an dieser Stelle auf die dortige Offenbarung hingewiesen werden kann.

Über eine manuelle Betätigung eines Griffbereichs 21 am proximalen Ende jedes Instrumententeils 2, 5 wird eine Verschwenkung der Instrumententeile 2, 5 initiiert, die eine entsprechende Bewegung eines Wirk-/Eingriffsbereichs (Branchen) 22 am distalen Ende jedes Instrumententeils 2, 5 (auch als Maulteil bezeichnet) nach sich zieht. Eine Instrumenten-Längsachse 11 verläuft längs des geschlossenen Instruments (Konstruktionslage) sowie eine Scharnierachse gemäß nachfolgender Beschreibung schneidend und dient im Rahmen dieser Anmeldung als Orientierung. Sie verläuft von einem proximalen Ende, an dem der Griffbereich 21 ausgebildet ist, zu einem distalen Ende, an dem der Wirkbereich (Branchen) 22 ausgebildet ist. Der Wirkbereich 22 ist nicht auf die in Fig. 1a gezeigte Klemmenform (Fasszange) beschränkt. Vielmehr kann er sämtliche Geometrien annehmen, um eine chirurgische Funktion zu erfüllen einschließlich Schneidklingen, Haken und dergleichen Maulteile.

Fig. 1b stellt das Instrument 1 aus Fig. 1a in einer Explosionsdarstellung dar. Eine Materialabflachung 16 ist im Bereich eines mittleren Führungsabschnitts 6 des männlichen Instrumententeils 5 ausgebildet, welche den Durchsteckabschnitt für das kastenförmige Hohlvolumen des weiblichen Instrumententeils 2 definiert. Fig. 1b stellt die Materialabflachung 16 in einer bevorzugten Ausführungsform dar. Hierbei verlaufen an der Oberseite des männlichen Instrumententeils 2 in deren axialem Mittelabschnitt zwei diametral voneinander abgewandte Einfräsungen parallel zueinander sowie quer zur Längsachse 11, wodurch sich am Rand jeder Einfräsung zwei in Instrumentenlängsrichtung beabstandete Schwenkbegrenzungskanten ausbilden, die, wie aus der Fig. 1a zu erkennen ist, mit dem kastenförmigen Hohlvolumen 4 zusammenwirken, um z.B. eine maximal geschlossene Position des chirurgischen Instruments zu definieren.

Die Materialabflachung 16 (d.h. die zwei Einfräsungen) bildet ferner in einem Bereich nahe einem die beiden Instrumententeile 2, 5 schwenkbar koppelnden Schwenkelement 7, wie etwa Schwenkbolzen, eine zusätzliche Vertiefung oder Senke 16a und in zwei Bereichen, in Instrumentenlängsrichtung gesehen proximal sowie distal entfernt vom Schwenkelement 8 jeweils eine Erhebung 16b, 16c, die durch die dazwischen ausgebildete Senke 16a voneinander in Instrumentenlängsrichtung beabstandet sind und die jeweils eine Führungs-/Gleitanlagefläche definieren. Mit der hinteren Führungsfläche 16b wird jener Bereich des Führungsabschnitts 6 bezeichnet, der proximal von der Vertiefung/Senke 16a liegt. Mit der vorderen Führungsfläche 16c wird jener Bereich des Führungsabschnitts 6 bezeichnet, der distal von der Vertiefung/Senke 16a liegt. Die Flächengrößenverhältnisse zwischen der hinteren Führungsfläche 16b, der Vertiefung/Senke 16a und der vorderen Führungsfläche 16c sind derart bestimmt, dass eine robuste Führung der Instrumententeile 2, 5 zueinander ermöglicht ist, während zugleich die erfindungsgemäßen Vorteile gelten, d.h. geringe Gleitreibungskräfte bei guter Bedienbarkeit.

Die vordere Führungsfläche 16c und die hintere Führungsfläche 16b sind in etwa gleich groß ausgestaltet. Die nach oben zeigenden Oberflächen der hinteren Führungsfläche 16b und der vorderen Führungsfläche 16c sind dazu vorbereitet, eine obere Innenfläche 14 des Hohlvolumens 4 gleit-zu-kontaktieren. Die nach unten zeigenden Oberflächen der hinteren Führungsfläche 16b und der vorderen Führungsfläche 16c sind dazu vorbereitet, eine untere Innenfläche 15 des Hohlvolumens 4 gleit-zu-kontaktieren.

Mittels dieser Gleitkontakte an den vorderen und hinteren Führungsflächen 16b, 16c ist bereits eine zuverlässige und gleichmäßige Gleitführung der Instrumententeile 2, 5 zueinander realisiert. Darüber hinaus bildet die Vertiefung/Senke 16a zusammen mit den oberen und unteren Innenflächen 14, 15 des kastenförmigen Hohlvolumens 4 an jeder der zwei Ausfräsungen einen Spalt vorbestimmter Spaltbreite aus, über den Desinfektionsmittel eingebracht werden kann, das sich dann auf die vorderen und hinteren Führungsflächen 16b, 16c ausbreitet. Die Senkentiefe gegenüber den Erhebungen 16b, 16c ist vorzugsweise so bemessen, dass sich das eingebrachte Desinfektionsmittel in dem vorstehend genannten Spalt gut ausbreiten kann.

Als obere Innenfläche 14 wird dabei die nach innen zeigende obere Oberfläche des Hohlvolumens 4 bezeichnet. Die untere Innenfläche 15 liegt der oberen Innenfläche 14 diametral gegenüber. Sowohl das kastenförmige Hohlvolumen 4 als auch der Führungsabschnitt (Durchsteckabschnitt) 6 ist mit einer zentralen Durchgangsbohrung versehen, die sich bei korrektem Durchstecken des Führungsabschnitts 6 durch das kastenförmige Hohlvolumen 4 überlappen, sodass das Schwenkelement 7 (Schwenkbolzen) für die Schwenkkopplung der beiden Instrumententeile 2, 5 hindurchgesteckt werden kann.

Fig. 2 zeigt eine vergrößerte Ansicht des Koppel-/Scharnierbereichs 3, wobei das weibliche Instrumententeil 2 aus Übersichtlichkeitsgründen hier nicht dargestellt ist. Das in Fig. 2 gezeigte männliche Instrumententeil 5 bildet mittels der Vertiefung/Senke 16a den vorstehend angedeuteten oberen/unteren Spalt 17, 18 zwischen der Vertiefung/Senke 16a an jeder der zwei Ausfräsungen und der oberen/unteren Innenfläche 14, 15 des Hohlvolumens 4 aus. Die hintere Führungsfläche 16b weist eine polygonartige Grundfläche auf. Vorzugsweise handelt es sich hierbei um ein Fünfeck / Pentagon mit einer rechteckigen Grundfläche. Die vordere Führungsfläche 16c weist im Wesentlichen die gleiche Form mit gespiegelter Orientierung auf. Die rechteckige Grundfläche schließt sich demnach an der Einfräskante der Vertiefung/Senke 16 unmittelbar an.

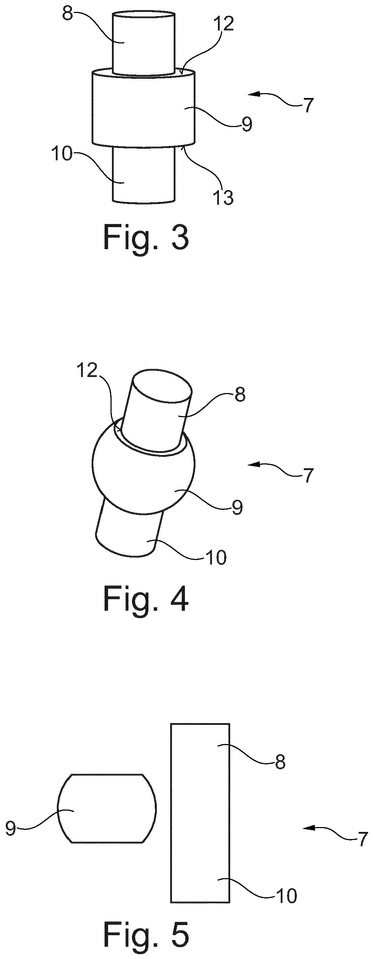

In Fig. 3 ist eine konkrete Ausführungsform eines Schwenkelements 7 gemäß dem bevorzugten Ausführungsbeispiel der Erfindung dargestellt. Dieses kann in dieser Ausführungsform einteilig oder aber auch zweiteilig ausgestaltet, wie aus der Explosionsdarstellung gemäß der Fig. 5 erkenntlich ist. Das Schwenkelement 7 ist in jedem Fall rotationssymmetrisch ausgestaltet und formschlüssig mit dem weiblichen und dem männlichen Instrumententeil 2, 5 verbunden/verbindbar. Auf diese Weise ist eine Verschwenkung der beiden Instrumententeile 2, 5 um eine Rotationsachse des Schwenkelements 7 realisierbar.

Im Konkreten ist das Schwenkelement 7 vorliegend der Schwenkbolzen mit drei axial unterteilten Abschnitten, nämlich einem axialen Mittenabschnitt 9 mit aufgeweitetem Durchmesser und zwei axialen Endabschnitten 8, 10 (zu beiden Seiten des Mittenabschnitts 9) mit vergleichsweise eingeschnürten Durchmessern. D.h., die Gliederung in die drei Bereiche, oberen Abschnitt 8, den Mittelabschnitt 9 und einen unteren Abschnitt 10 zeichnet sich in diesem Fall durch unterschiedliche Durchmesser aus. Der Durchmesser des oberen und des unteren Abschnitts 7, 10 ist kleiner ausgestaltet als der des Mittelabschnitts 10.

Das vorliegende Schwenkelement 7 gemäß der Fig. 3 ist einteilig ausgestaltet. Erfindungsgemäß bildet sich infolge der Durchmesserunterschiede zwischen dem oberen Abschnitt 8 und dem Mittelabschnitt 9 in Radialrichtung eine umlaufende Stufe/Absatz. Diese definiert eine obere Anlagestufe (oberer Anschlag, obere Auflagekante) 12 aus. Die obere Anlagestufe 12 ist dazu vorbereitet, im montierten Zustand des Schwenkbolzens 7 das Hohlvolumen 4 der weiblichen Branche 2 zu kontaktieren. Aus diesem Grund ist die obere Anlagestufe 12 besonders glatt und eben ausgestaltet.

Die grundsätzliche Form des Mittelabschnitts 9 gemäß Fig. 3 ist in der vorliegenden Ausführungsform eine zylindrische. Der Mittelabschnitt 9, der in dieser und in den anderen Ausführungsformen im Wesentlichen eine vergleichbare Länge entlang seiner Rotationsachse aufweist, wie der obere Abschnitt 8 und wie der untere Abschnitt 10, weist, wie vorstehend erwähnt, einen größeren Durchmesser auf als der obere und der untere Abschnitt 8, 10 auf. Der Unterschied jener Durchmesser liegt in dem Bereich zwischen Faktor 1,2 und 2,5.

Folgerichtig bildet der Mittelabschnitt 9 und der untere Abschnitt 10 ebenfalls eine Anlagestufe (unterer Anschlag, untere Auflagekante) 13 aus, wie diese vorstehend bereits anhand der Stufe 12 beschrieben wurde. Die Stufe 13 zwischen dem unteren Abschnitt 10 und dem Mittelabschnitt 9 wird demnach als untere Anlagestufe 13 bezeichnet. Diese weist, analog zur oberen Stufe 12, die Funktion auf, das Hohlvolumen 4 der weiblichen Branche 2 im zusammengebauten Zustand 2 zu kontaktieren. Die untere Stufe 13 und die obere Stufe 12 weisen eine identische Form auf und sind zueinander planparallel.

Fig. 4 stellt das Schwenkelement 7 in einer weiteren einteiligen Ausführungsform der vorliegenden Erfindung dar. Hierbei ist ebenso eine obere Anlagestufe 12 und eine untere Anlagestufe 13 ausgebildet. Die Form des Mittelabschnitts 9 ist zwischen der oberen Stufe 12 und der unteren Stufe 13 in der vorliegenden Ausführungsform jedoch im Unterschied zur Ausführungsform gemäß Fig. 3 ballig (radial-konvex) ausgestaltet. In diesem Abschnitt weist der Mittelabschnitt 9 demnach eine Kugelform auf.

Für beide Ausführungsformen gemäß der Fig. 3 und 4 ist es aber vorgesehen, dass der axiale Abstand zwischen der oberen und unteren Anlagestufe 12, 13 dem Parallelabstand der oberen und unteren Innenflächen 14, 15 des kastenförmigen Hohlvolumens 4 des weiblichen Instrumententeils 2 entspricht. Durch die Gleichsetzung dieser beiden Distanzen ist eine gleichmäßige Führung des weiblichen Instrumententeils 2 relativ zum männlichen Instrumententeil 5 sichergestellt, da sämtliche Anlageflächen (obere und untere Innenflächen 14, 15) des Hohlvolumens 4 sowohl mit den Gleitflächen an den Erhebungen 16b, 16c der männlichen Instrumententeil 5 wie auch mit dem Anlagestufen 12, 13 des Schwenkstifts in Gleitanlage kommen.

Analog zum oberen Spalt 17 gibt es einen unteren Spalt 18, wie dies in Fig. 2 deutlich zu ersehen ist. Für diesen gelten die gleichen Eigenschaften wie für den oberen Spalt 17, nur dass er in einer entgegengesetzten Richtung auf einer unteren Oberfläche 16a der Vertiefung/Senke 16a ausgestaltet ist.

In der vorliegenden Ausführungsform ist der Mittelabschnitt 9 des Schwenkelements 7 zwischen der oberen Anlagestufe 12 und der unteren Anlagestufe 13 vorzugsweise kugelförmig / sphärisch ausgestaltet. Auf diese Weise ist eine Art Kugelgelenklagerung des Schwenkelements 7 in dem Führungsabschnitt 6 des männlichen Instrumententeils 5 realisiert. Aufgrund der Vertiefung/Senke 16a ist daher in einer bestimmten Relativdrehlage beider Instrumententeile 2, 5 eine dreidimensionale Bewegung/Verschwenkung/Kippung des weiblichen Instrumententeils 2 relativ zum männlichen Instrumententeil 5 ermöglicht. Diese Verschwenkung/Kippung dient hauptsächlich der erhöhten Desinfizierbarkeit / Reinigbarkeit des chirurgischen Instruments 1 insbesondere im Scharnierbereich 3 beider Instrumententeile 2, 5. Weiterhin erhöht sie ihre Flexibilität.

Das einteilige Schwenkelement 7 ist beispielsweise aus X40Cr 13 herstellbar und härtbar. Dadurch, dass das Schwenkelement 7 aus einem anderen Material gefertigt ist als die Instrumententeile 2, 5, folgt, dass ein Festfressen der beiden Instrumententeile 2, 5 zusätzlich erschwert wird.

Eine weitere Ausführungsform des Schwenkelements 7 ist in Fig. 5 dargestellt. Hierbei handelt es sich um ein zweiteiliges Schwenkelement 7. Zweiteilig bedeutet, dass sich das Schwenkelement 7 aus einem Stift/Bolzen und aus einem Ring/Hülse zusammensetzt, der auf den (Scharnier-)Stift aufgezogen wird. Der Stift bildet hierbei an seinem oberen Ende den oberen Abschnitt 8 und an seinem unteren Ende den unteren Abschnitt 10 aus. Der Bereich des Stiftes zwischen dem oberen und dem unteren Abschnitt 8, 10, ist dazu vorbereitet, den Ring/Hülse aufzunehmen, wodurch sich funktional der Mittelabschnitt 9 ausbildet. Hinsichtlich ihrer Geometrie weist die zweiteilige Ausgestaltung des Schwenkelements 7 keine Unterschiede zur einteiligen Ausgestaltung auf. Dafür ist es auf diese Weise möglich, den Ring und den Stift aus zwei unterschiedlichen Materialien zu fertigen. Beispielhaft sei hierzu die Kombination Austernit und Keramik angeführt.

Die Geometrie des Schwenkelements 7 ist frei wählbar. Erfindungsgemäß sind aber die obere und die untere Stufe 12, 13 erforderlich, um die Vorteile der stabilen Schwenkführung zu erhalten. Die restliche Ausgestaltung des Mittelabschnitts 9 oder auch des unteren Abschnitts 10 und des oberen Abschnitts 8 ist je nach Anwendungsfall jedoch variierbar, um das Schwenkelement 7 an die vorherrschenden Bedingungen optimal anzupassen.

### Bezugszeichenliste

- 1.: Chirurgisches Instrument
- 2.: weibliches Instrumententeil
- 3.: Koppelbereich
- 4.: Hohlvolumen
- 5.: männliches Instrumententeil
- 6.: Führungsabschnitt
- 7.: Schwenkelement
- 8.: oberer Abschnitt
- 9.: Mittelabschnitt
- 10.: unterer Abschnitt
- 11.: Längsachse
- 12.: obere Anlagestufe / Auflagekante
- 13.: untere Anlagestufe / Auflagekante
- 14.: obere Innenfläche des Hohlvolumens
- 15.: untere Innenfläche des Hohlvolumens
- 16.: Ausfräsung(en)
- 16a.: Vertiefung(en)/Senke(n)
- 16b.: hintere Führungsfläche
- 16c.: vordere Führungsfläche
- 17.: oberer Spalt
- 18.: unterer Spalt
- 19.: obere Oberfläche (obere Abflachung)
- 20.: untere Oberfläche (untere Abflachung)
- 21.: Griffbereich
- 22.: Wirkbereich/Maulteil

## Patentansprüche

1. Chirurgisches Instrument (1) mit zwei scharnierartig miteinander gekoppelten Instrumententeilen (2, 5), die in ihrem Kopplungsbereich (3) jeweils zumindest eine Anlageseite (14, 15, 16) ausbilden, an denen die beiden Instrumententeile (2, 5) um einen im Kopplungsbereich (3) angeordneten oder ausgebildeten Scharnierbolzen (7) schwenkgleitend aneinanderliegen, wobei zumindest eine Anlageseite (16) der einen oder beider Instrumententeile (2, 5) in einem scharnierbolzennahen Bereich eine oberflächige Materialabtragung aufweist, wodurch sich in diesem Bereich eine Senke (16a) mit vorbestimmter Senktiefe und in Instrumentenlängsrichtung beidseits des Scharnierbolzens (7) in scharnierbolzenentfernten Bereichen der Anlageseite (16) jeweils eine Erhebung (16b, 16c) ausbilden, welche die Senke (16a) in Instrumentenlängsrichtung zwischen sich einschließen, **dadurch gekennzeichnet, dass** der Scharnierbolzen (7) in Konstruktionslage zumindest eine in dessen Axialrichtung wirkende Auflagekante (12, 13) in Höhe der Erhebung (16b, 16c) definiert.

2. Chirurgisches Instrument (1) nach Anspruch 1, **dadurch gekennzeichnet, dass** durch die Ausbildung der zumindest zwei Erhebungen (16b, 16c) an dem einen Instrumententeil (5) sowie der zumindest einen Auflagekante (12, 13) an dem Scharnierbolzen (7) eine Art Drei-Punkt-Gleitlagerung für das gegenüberliegende, anscharnierte Instrumententeil (2) geschaffen wird.

3. Chirurgisches Instrument (1) nach einem der vorstehenden Ansprüche, **dadurch gekennzeichnet, dass** die zumindest eine scharnierbolzenseitige Auflagekante (12, 13) durch einen gegenüber dem zumindest einen axialen Endabschnitt (8) verdickten oder radial aufgeweiteten Mittelabschnitt (9) in stoffeinstückiger Bauweise oder durch eine auf den Scharnierbolzen (7) aufgeschobene Hülse (9) in zweiteiliger Bauweise ausgebildet ist.

4. Chirurgisches Instrument nach Anspruch 3, **dadurch gekennzeichnet, dass** der radial aufgeweitete Mittelabschnitt (9) oder die aufgeschobene Hülse (9) mantelseitig eine ballige, vorzugsweise sphärische Kontur hat, derart, dass der Scharnierkontakt zwischen dem einen, vorzugsweise männlichen Instrumententeil (5) und dem Scharnierbolzen (7) linienförmig ist.

5. Chirurgisches Instrument (1) nach einem der vorstehenden Ansprüche, **dadurch gekennzeichnet, dass** das eine Instrumententeil (2) als das weibliche Instrumententeil im Scharnierbereich (3) einen Durchsteckkasten ausformt mit zwei diametral einander zugewandten vorzugsweise ebenen inneren Anlageseiten und das andere Instrumententeil (5) als das männliche Instrumententeil im Scharnierbereich (3) einen Durchsteckabschnitt ausformt mit zwei diametral voneinander abgewandten äußeren Anlageseiten, die in Gleitkontakt mit den inneren Anlageseiten des weiblichen Instrumententeils (2) stehen und beide äußeren Anlageseiten des männlichen Instrumententeils (5) jeweils eine scharnierbolzennahe Senke sowie zwei scharnierbolzenferne Erhebungen aufweisen.

6. Chirurgisches Instrument (1) nach Anspruch 5, **dadurch gekennzeichnet, dass** der Scharnierbolzen (7) in Konstruktionslage zwei axialbeabstandete Anlagekanten (12, 13) in Höhe der jeweiligen Erhebungen definiert, an denen die jeweils gegenüberliegende innere Anlageseite des weiblichen Instrumententeils (2) schwenkgleitend anliegen.

7. Chirurgisches Instrument (1) nach Anspruch 1 **dadurch gekennzeichnet, dass** die zumindest eine Auflagekante (12, 13) dazu vorbereitet ist, die zumindest eine Anlageseite (14, 15) des zumindest einen Instrumententeils (2, 5) zu kontaktieren.

## Claims

1. Surgical instrument (1) with two instrument parts (2, 5) coupled together by means of a hinge, which form at least one attachment site (14, 15, 16) in their coupling area (3) where the two instrument parts (2, 5) are aligned to pivot about a hinge pin (7) that is arranged or designed in the coupling area (3), wherein at least one attachment site (16) of the one or both instrument parts (2, 5) has a section of surface material removed in the area of a hinge pin groove, whereby in this region a recess (16a) with a predetermined depression depth and arranged longitudinally on the instrument results in an elevation (16b, 16c) on both sides of the hinge pin (7) in the regions of the attachment site (16) remote from the hinge pin, which enclose the recess (16a) between themselves longitudinally along the instrument, **characterised in that** the hinge pin (7) features a design attitude that defines at least one supporting edge (12, 13) at the height of the elevation (16b, 16c), which operates in its axial direction.

2. Surgical instrument (1) according to claim 1, **characterised in that** a kind of three-point slide bearing is created for the hinged instrument part opposite (2) by the formation of the at least two elevations (16b, 16c) and the one instrument part (5) and the at least one supporting edge (12, 13) on the hinge pin (7).

3. Surgical instrument (1) according to one of the preceding claims, **characterised in that** the at least one supporting edge (12, 13) on the side of a hinge pin is formed by a thickened or radially expanded central section (9) opposite the at least one axial end section (8) in monobloc material construction, or in a two piece design by a protracted sleeve (9) on the hinge pin (7).

4. Surgical instrument according to claim 3, **characterised in that** the radially expanded central section (9) or the protracted sleeve (9) has a convex, preferably spherical, contour on the cover side, such that the hinge contact between the one, preferably male instrument part (5) and the hinge pin (7) is linear.

5. Surgical instrument (1) according to one of the preceding claims, **characterised in that** the one instrument part (2) being the female instrument part forms an insertion case in the hinge section (3) with two diametrically facing preferably planar inner attachment sites, and the other instrument part (5) being the male instrument part forms an insertion segment in the hinge section (3) with two exterior attachment sites diametrically opposite each other and averted, which are in sliding contact with the inner attachment sites of the female instrument part (2) and both outer attachment sites of the male instrument part (5), which each have a recess adjacent to the hinge pin as well as two elevations remote from the hinge pin.

6. Surgical instrument (1) according to claim 5, **characterised in that** the design attitude of the hinge pin (7) defines two axially spaced supporting edges (12, 13) at the level of the respective elevations, on which the respective opposite inner attachment site of the female instrument part (2) is attached.

7. Surgical instrument (1) according to claim 1, **characterised in that** the at least one supporting edge (12, 13) is designed to make contact with the at least one attachment site (14, 15) of the at least one instrument part (2, 5).

## Revendications

1. Instrument (1) chirurgical avec deux parties d'instrument (2, 5) couplées l'une à l'autre à la manière d'une charnière, qui forment dans leur zone de couplage (3) respectivement au moins un côté d'appui (14, 15, 16), au niveau desquels les deux parties d'instrument (2, 5) reposent l'une au niveau de l'autre avec un glissement par pivotement autour d'un axe de charnière (7) disposé ou réalisé dans la zone de couplage (3), dans lequel au moins un côté d'appui (16) de l'une ou des deux parties d'instrument (2, 5) présente un enlèvement de matériau en surface dans une zone proche de l'axe de charnière, ce qui a pour effet que se forment dans cette zone un creux (16a) avec une profondeur de creux prédéfinie et, dans le sens longitudinal d'instrument, de part et d'autre de l'axe de charnière (7), dans des zones éloignées de l'axe de charnière du côté d'appui (16), respectivement une partie surélevée (16b, 16c), lesquelles renferment entre elles le creux (16a) dans le sens longitudinal d'instrument, **caractérisé en ce que** l'axe de charnière (7) définit en position de construction au moins une arête de support (12, 13) agissant dans sa direction axiale à hauteur de la partie surélevée (16b, 16c).

2. Instrument (1) chirurgical selon la revendication 1, **caractérisé en ce qu'**une sorte de palier lisse à trois points pour la partie d'instrument (2) faisant face montée sur charnières est créé par la formation des au moins deux parties surélevées (16b, 16c) au niveau d'une partie d'instrument (5) ainsi qu'au niveau de l'au moins une arête de support (12, 13) au niveau de l'axe de charnière (7).

3. Instrument (1) chirurgical selon l'une quelconque des revendications précédentes, **caractérisé en ce que** l'au moins une arête de support (12, 13) côté axe de charnière est réalisée par une section centrale (9) épaissie ou radialement élargie par rapport à l'au moins une section d'extrémité (8) axiale dans une conception en continuité de matériau ou par une douille (9) enfilée sur l'axe de charnière (7) dans une conception en deux parties.

4. Instrument chirurgical selon la revendication 3, **caractérisé en ce que** la section centrale (9) radialement élargie ou la douille (9) enfilée a, côté extérieur, un contour bombé, de préférence sphérique de telle manière que le contact de charnière est de forme linéaire entre une partie d'instrument (5) de préférence mâle et l'axe de charnière (7).

5. Instrument (1) chirurgical selon l'une quelconque des revendications précédentes, **caractérisé en ce qu'**une partie d'instrument (2) forme un boîtier traversant en tant que la partie d'instrument femelle dans la zone de charnière (3), avec deux côtés d'appui intérieurs de préférence plats tournés l'un vers l'autre diamétralement et l'autre partie d'instrument (5) forme une section traversante en tant que la partie d'instrument mâle dans la zone de charnière (3), avec deux côtés d'appui extérieurs diamétralement opposés l'un à l'autre, qui se trouvent en contact avec glissement avec les côtés d'appui intérieurs de la partie d'instrument (2) femelle, et les deux côtés d'appui extérieurs de la partie d'instrument (5) mâle présentent respectivement un creux proche de l'axe de charnière ainsi que deux parties surélevées éloignées de l'axe de charnière.

6. Instrument (1) chirurgical selon la revendication 5, **caractérisé en ce que** l'axe de charnière (7) définit en position de construction deux arêtes d'appui (12, 13) espacées axialement à hauteur des parties surélevées respectives, au niveau desquelles le côté d'appui intérieur faisant face respectivement de la partie d'instrument (2) femelle repose avec glissement par pivotement.

7. Instrument (1) chirurgical selon la revendication 1, **caractérisé en ce que** l'au moins une arête de support (12, 13) est préparée pour établir un contact avec l'au moins un côté d'appui (14, 15) de l'au moins une partie d'instrument (2, 5).
